# EUROPÄISCHE PATENTSCHRIFT

(11) **EP 0 775 687 B1**
(45) Veröffentlichungstag und Bekanntmachung des Hinweises auf die Patenterteilung: **21.02.2001**
(21) Anmeldenummer: 96118091.6
(22) Anmeldetag: 12.11.1996
(51) Int. Cl.: C07C 67/62, C07C 67/08, C07C 69/54

(54) **Verfahren zur Herstellung von (Meth)acrylsäureestern**
Process for the preparation of esters of (meth)acrylic acid
Procédé de préparation d'esters d'acide (méth)acrylique

(30) Priorität: 22.11.1995 DE 19543464
(43) Veröffentlichungstag der Anmeldung: 28.05.1997
(73) Patentinhaber: BASF AKTIENGESELLSCHAFT, 67056 Ludwigshafen (DE)
(72) Erfinder: Häussling, Lukas, 67098 Bad Dürkheim (DE); Bernhard, Ludwig, 64646 Heppenheim (DE); Reich, Wolfgang, 67133 Maxdorf (DE); Schwalm, Reinhold, 67157 Wachenheim (DE); Beck, Erich, 68258 Ladenburg (DE)

(56) Entgegenhaltungen:
- EP-A- 0 680 985
- DATABASE WPI Section Ch, Week 9436 Derwent Publications Ltd., London, GB; Class A41, AN 94-290862 XP002024873 & JP 06 219 991 A (ARAKAWA CHEM IND LTD) , 9.August 1994

## Beschreibung

Die Erfindung betrifft ein Verfahren zur Herstellung von (Meth)acrylsäureestern. Es sind unterschiedliche Verfahren zur Herstellung von (Meth)acrylsäureestern bekannt. All diesen Verfahren ist gemeinsam, daß überschüssige bzw. nicht umgesetzte (Meth)acrylsäure aus dem Reaktionsprodukt entfernt wird. Ein Gehalt an (Meth)acrylsäure ist insbesondere aufgrund des stechenden Geruchs dieser Verbindung bei der späteren Verwendung der (Meth)acrylsäureester unerwünscht.

Nach dem Verfahren der DE-A-2913218 werden flüchtige Bestandteile nach Herstellung der (Meth)acrylsäureester durch Einblasen von Luft entfernt. In der EP-A-618187 wird ein Auswaschverfahren zur Entfernung der (Meth)acrylsäure beschrieben. Eine andere Möglichkeit zur Entfernung der restlichen (Meth)acrylsäure ist die Umsetzung mit Epoxiden zu Epoxyacrylaten, wie es z.B. aus EP-A-127766 oder auch EP-A-0 680 985 bekannt ist.

Aus JP 6 219 991 (Database WPI Section Ch, Week 9436, Class A41, AN-94-290862) ist die Neutralisierung der restlichen (Meth)acrylsäure mit Alkalihydroxyden bekannt.

Primäre oder sekundäre Aminoverbindungen erhöhen bekannterweise die Reaktivität von (Meth)acrylsäureestern bei der Strahlungshärtung. (Meth)acrylsäureester werden daher z.B. gemäß EP-A-586849 und EP 280222 für die Verwendung in strahlungshärtbaren Beschichtungen mit primären oder sekundären Aminoverbindungen modifiziert.

Aufgabe der vorliegenden Erfindung war daher ein Verfahren zur Herstellung von (Meth)acrylsäureestern, bei dem auf eine nahezu vollständige Abtrennung der überschüssigen oder nicht umgesetzten (Meth)acrylsäure verzichtet werden kann und die erhaltenen (Meth)acrylsäureester sich gut zur Verwendung als oder in strahlungshärtbaren Massen eignen.

Demgemäß wurde ein Verfahren zur Herstellung von (Meth)acrylsäureestern durch Veresterung von (Meth)acrylsäure mit ein- oder mehrwertigen Alkoholen, dadurch gekennzeichnet, daß keine Umsetzung der restlichen (Meth)acrylsäure mit Epoxiden erfolgt, sondern daß dem Reaktionsgemisch, welches noch (Meth)acrylsäure entsprechend einer Säurezahl von mindestens 5 mg KOH pro 1 g Reaktionsgemisch enthält, mindestens eine Aminoverbindung mit mindestens einer primären, sekundären oder tertiären Aminogruppe, welche mit (Meth)acrylsäure ein Salz bildet, zugesetzt wird, gefunden.

Die Veresterung von Methacrylsäure oder vorzugsweise Acrylsäure mit ein- oder vorzugsweise mehrwertigen Alkoholen ist als solche bekannt.

Als mehrwertige Alkohole zu nennen sind insbesondere C₂ - C₁₀ Alkandiole, -triole oder -tetrole, z.B. Butandiol, Hexandiol, Trimethylolpropan, Pentaerythrit.

In Betracht kommen weiterhin auch Hydroxylgruppen enthaltende Ether bzw. Polyether oder Hydroxylgruppen enthaltende Polyester. Vorzugsweise handelt es sich dabei um Ether, insbesondere Polyether und Polyester mit 2 bis 6, vorzugsweise 2 bis 4 und besonders bevorzugt 2 Hydroxylgruppen.

Die Molekulargewichte Mₙ der Polyester bzw. Polyether oder Ether liegen dabei bevorzugt zwischen 100 und 4000.

Derartige hydroxylgruppenhaltige Polyester können z.B. in üblicher Weise durch Veresterung von Dicarbonsäuren oder Polycarbonsäuren mit Diolen oder Polyolen hergestellt werden. Die Ausgangsstoffe für solche hydroxylgruppenhaltige Polyester sind dem Fachmann bekannt. Bevorzugt können als Dicarbonsäuren Bernsteinsäure, Glutarsäure, Adipinsäure, Sebacinsäure, o-Phthalsäure, deren Isomere und Hydrierungsprodukte sowie veresterbare Derivate, wie Anhydride, z.B. Maleinsäureanhydrid oder Dialkylester der genannten Säuren eingesetzt werden. Als Polycarbonsäure bzw. deren Anhydride seien Tri- oder Tetrasäuren wie Trimellitsäureanhydrid oder Benzoltetracarbonsäure genannt. Als Diole kommen vorzugsweise in Betracht Ethylenglykol, Propylenglykol-1,2 und -1,3, Butandiol-1,4, Hexandiol-1,6, Neopentylglykol, Cyclohexandimethanol sowie Polyglykole vom Typ des Ethylenglykols und Propylenglykols.

Als Polyole sind in erster Linie Trimethylolpropan, Glycerin oder Pentaerythrit sowie Ditrimethylolpropan, Sorbit und Dipentaerythrit zu nennen.

Als Diole oder Polyole in Betracht kommen auch oxalkylierte (z.B. mit Ethylenoxid oder Propylenoxid) Diole oder Polyole, insbesondere mit einem Oxalkylierungsgrad von 0 bis 10, bezogen auf die jeweiligen Hydroxygruppen des Diols oder Polyols.

Als hydroxylgruppenhaltige Polyether kommen z.B. solche in Frage, welche nach bekannten Verfahren durch Umsetzung von zwei- und/oder mehrwertigen Alkoholen mit verschiedenen Mengen an Ethylenoxid und/oder Propylenoxid erhalten werden können. Desgleichen sind auch Polymerisationsprodukte des Tetrahydrofurans oder Butylenoxids verwendbar.

Bevorzugt sind Oxalkylierungsprodukte der obengenannten Diole oder Polyole, insbesondere mit einem Oxalkylierungsgrad von 0 bis 10, bezogen auf die jeweiligen Hydroxylgruppen des Diols oder Polyols, wobei mindestens 2 Ethergruppen im Molekül enthalten sind.

Bei der Veresterung kann die Acrylsäure bzw. Methacrylsäure in Unterschuß oder auch in großem Überschuß, bezogen auf die OH-Gruppen, eingesetzt werden.

Bevorzugt werden 0,7 bis 2,0 Mol, besonders bevorzugt 0,9 bis 1,5 Mol und ganz besonders bevorzugt 1,0 bis 1,5 Mol Acrylsäure bzw. Methacrylsäure auf ein Mol OH-Gruppen der zu veresternden Alkohole eingesetzt.

Bei der Veresterung wird vorzugsweise ein Veresterungskatalysator, z.B. Schwefelsäure oder p-Toluolsulforsäure in Mengen von vorzugsweise 0,1 bis 3 Gew.-% bezogen auf die Gewichtssumme der zu veresternden Komponenten verwendet.

Zur Vermeidung einer Polymerisation der (Meth)acrylsäure werden üblicherweise auch Polymerisationsinhibitoren zugegeben.

Die Veresterung erfolgt z.B. bei 40 - 120°C, vorzugsweise 70 - 120°C, wobei sich als Schleppmittel für die Entfernung des Reaktionswassers aliphatische oder cycloaliphatische oder aromatische Kohlenwasserstoffe oder Gemische derselben, insbesondere solche mit einem Siedebereich zwischen 40 und 120°C, vorzugsweise zwischen 70 und 120°C, als besonders geeignet erwiesen haben.

Geeignete aliphatische Kohlenwasserstoffe sind beispielsweise Hexan und seine Isomeren, Cyclohexan, Methylcyclohexan, vor allem aber höhersiedende Kohlenwasserstoffgemische, die auch höher- oder niedersiedende Kohlenwasserstoffe enthalten können. Besonders bevorzugt ist Toluol. Die Menge des zugefügten Kohlenwasserstoffs ist in keiner Weise kritisch; je nach den verwendeten Apparaturen, in denen die Veresterung durchgeführt wird, kann die zugesetzte Menge zwischen der 0,05- und 2-fachen Menge des Reaktionsgemisches aus (Meth)acrylsäure und Alkohol gewählt werden. Vorteilhaft ist ein Mengenverhältnis von 1 : 0,1 bis 1 : 0,5 Veresterungsgemisch zu Kohlenwasserstoff.

Das Kohlenwasserstoff-Lösungsmittel dient beim Vorgang der Veresterung als Schleppmittel, um das freigesetzte Wasser auszukreisen. Die Veresterung im Sinne der Erfindung muß also unter apparativen Bedingungen durchgeführt werden, bei denen ein solches Auskreisen, d.h., ein Abscheiden des Reaktionswassers, möglich ist. Gewöhnlich bedient man sich hierbei der üblichen Wasserabscheider. Der Reaktionsablauf kann z.B. durch Bestimmen der Säurezahl verfolgt werden.

Im allgemeinen wird die Veresterung abgebrochen, sobald eine Säurezahl von 40 bis 120 mg KOH pro 1 g Reaktionsgemisch (Summe aus Reaktionsprodukt und restlichen Ausgangskomponenten, ohne Lösungsmittel, Wasser) erreicht ist. Die Säurezahl wird nach DIN 53402 bestimmt.

Flüchtige Bestandteile, wie organische Lösungsmittel oder Wasser oder auch gegebenenfalls ein Teil der (Meth)acrylsäure, können aus dem Reaktionsgemisch z.B. durch Vakuumdestillation entfernt werden. Zur Entfernung der restlichen Acrylsäure oder Methacrylsäure werden im Stand der Technik verschiedene Verfahren vorgeschlagen, welche im einzelnen sehr aufwendig und mit Nachteilen verbunden sind (Siehe Einleitung).

Bei dem erfindungsgemäßen Verfahren wird nach Erreichen einer Säurezahl von mindestens 5 mg KOH pro 1 g Reaktionsgemisch, bevorzugt von 5 bis 120 mg KOH, besonders bevorzugt von 10 bis 80 und ganz besonders bevorzugt von 15 bis 40 mg KOH pro 1 g Reaktionsgemisch mindestens eine Aminoverbindung mit mindestens einer primären, sekundären oder tertiären Aminogruppe zugesetzt.

Die Menge der zugesetzten Aminoverbindung bzw. Aminoverbindungen wird vorzugsweise so bemessen, daß die molare Zahl der primären, sekundären und/oder tertiären Aminogruppen der Molzahl der noch vorhandenen Acrylsäure bzw. Methacrylsäure mindestens entspricht. Bevorzugt beträgt die Anzahl Mole der Aminogruppen zur Anzahl der Mole (Meth)acrylsäure 1:1 bis 10:1 und besonders bevorzugt 1:1 bis 5:1.

Die zugesetzten Aminoverbindungen bilden mit der (Meth)acrylsäure Salze, welche im Gegensatz zur freien (Meth)acrylsäure geruchsfrei sind. Soweit primäre oder sekundäre Aminogruppen vorhanden sind kommt es auch zum Teil zu einer Michael-Addition dieser Aminogruppen mit der Acrylsäure oder den erhaltenen Acrylsäureestern.

Bevorzugte Aminoverbindungen sind solche mit einem Molekulargewicht unter 2000 bevorzugt unter 1000, besonders bevorzugt unter 500 g/l mol.

Insbesondere bevorzugt sind Aminoverbindungen mit einer Dissoziationskonstanten pK_{b} < 3,5 besonders bevorzugt < 3 (in Wasser bei 25°C). Bei mehrstufiger Dissoziation gilt der angegebene Wert für die 1. Dissoziationsstufe.

Besonders bevorzugte Aminoverbindungen mit primären Aminogruppen sind
C₂ - C₁₀ Alkylamine, welche durch eine
   C₁ - C₁₀ Alkoxygruppe am Alkylrest substituiert sind,
z.B. 3 Methoxy-propylamin, 3 Ethoxypropylamin und 2 Ethylhexoxy 3-propylamin.

Die primären Aminogruppen, d.h. mit einem Substituenten, enthalten vorzugsweise ein Heteroatom, insbesondere ein Sauerstoffatom, in dem Substituenten. Vorzugsweise befindet sich das Heteroatom in γ- oder δ-Stellung zum Stickstoffatom, d.h. Stickstoffatom und Heteroatom sind über 2 bzw. 3 Kohlenstoffatome miteinander verbunden.

Besonders bevorzugte Aminoverbindungen mit sekundären Aminogruppen sind
N-Ethyl-isopropanolamin,
N-Ethyl-cyclohexylamin.

Bei den sekundären Aminogruppen (d.h. mit 2 Substituenten) sind die beiden Substituenten vorzugsweise ungleich. Insbesondere enthält mindestens einer der Substituenten ein Heteroatom wie vorstehend bei den primären Aminogruppen beschrieben.

Besonders bevorzugte Aminoverbindungen mit tertiären Aminogruppen sind
N,N-Dimethyl-isopropylamin,
N-Hydroxyethyl-morpholin
N-Hydroxyethyl, N,N dibutylamin
N-Methyl, N,N diisopropanolamin.

Bei den tertiären Aminogruppen (d.h. mit 3 Substituenten) sind vorzugsweise mindestens 2 Substituenten ungleich. Insbesondere enthält mindestens einer der Substituenten ein Heteroatom wie vorstehend bei den primären Aminogruppen beschrieben.

Besonders bevorzugte Aminoverbindungen mit primären und tertiären Aminogruppen sind
N,N-Diethyl-4 Amino-Pentylamin
NN, -Diethylamino-3 propylamin.

Für die Art der primären und tertiären Aminogruppen in diesen Aminoverbindungen gilt das voranstehend geschriebene.

Die Zugabe von Aminoverbindungen zu organischen Gemischen, welche Säuren, z.B. Acrylsäure, Methacrylsäure oder auch z.B. saure Veresterungskatalysatoren enthalten, kann mitunter zum Auskristallisieren der entstehenden Salze aus organischen Medien führen. Bei den vorstehenden bevorzugten und besonders bevorzugten Aminoverbindungen wurde nach Zugabe zu den (Meth)acrylsäureestern, welche die oben genannte Restmenge Acrylsäure oder Methacrylsäure und auch gegebenenfalls noch Restmengen des Veresterungskatalysators enthalten, kein Auskristallisieren von Salzen beobachtet. Auch über große Zeiträume z.B. von 3 Monaten waren die (Meth)acrylsäureester lagerstabil, d.h. Trübungen traten nicht auf.

Die nach den erfindungsgemäßen Verfahren erhältlichen (Meth)acrylsäureester können allein, d.h. als einzige strahlungshärtbare Verbindungen oder zusammen mit anderen strahlungshärtbaren Verbindungen in strahlungshärtbaren Massen Verwendung finden. Bevorzugte strahlungshärtbare Massen enthalten 10 bis 100 Gew.-%, besonders bevorzugt 50 bis 100 Gew.-%, ganz besonders bevorzugt 70 bis 100 Gew.-% der erfindungsgemäßen (Meth)acrylsäureester, bezogen auf die Gesamtmenge der strahlungshärtbaren Verbindungen.

Die strahlungshärtbaren Massen können als bzw. in Beschichtungsmassen, z.B. Lacken, Druckfarben oder Klebstoffen, als Druckplatten, als Formkörper, zur Herstellung von Photoresisten, in der Stereolithographie oder als Gießmasse, z.B. für optische Linsen verwendet werden.

Zur Verwendung als oder in strahlungshärtbaren Massen können Zusatzstoffe, z.B. Vernetzer, Verdicker, Verlaufsmittel oder Füllstoffe bzw. Pigmente etc. zugesetzt werden.

Die strahlungshärtbaren (Meth)acrylsäureester bzw. die strahlungshärtbaren Massen können thermisch, vorzugsweise durch energiereiche Strahlung wie UV-Licht oder Elektronenstrahlen gehärtet werden.

Zur Strahlungshärtung durch UV-Licht werden üblicherweise Photoinitiatoren zugesetzt.

Als Fotoinitiatoren in Betracht kommen z.B. Benzophenon und Derivate davon, wie z.B. Alkylbenzophenone, halogenmethylierte Benzophenone, Michlers Keton, sowie Benzoin und Benzoinether wie Ethylbenzoinether, Benzilketale wie Benzildimethylketal, Acetophenonderivate wie z.B. Hydroxy-2-methyl-phenylpropan-1-on und Hydroxycyclohexyl-phenylketon, Anthrachinon und seine Derivate wie Methylanthrachinon und insbesondere Arylphosphinoxide wie z.B. Lucirin^{®} TPO (2,4,6-Trimethylbenzoyldiphenylphosphinoxid).

Die Fotoinitiatoren, die je nach Verwendungszweck der erfindungsgemäßen Massen in Mengen zwischen 0,1 und 15 Gew.-%, vorzugsweise 1 bis 10 Gew.-%, bezogen auf die polymerisierbaren Komponenten, eingesetzt werden, können als einzelne Substanz oder, wegen häufiger vorteilhafter synergistischer Effekte, auch in Kombination miteinander verwendet werden.

Die strahlungshärtbaren (Meth)acrylsäureester zeigen gute anwendungstechnische Eigenschaften, z.B. Härte, Flexibilität, Chemikalienbeständigkeit und insbesondere eine verbesserte Haftung auf unterschiedlichsten Substraten, z.B. auf Glas.

### Beispiele:

### 7. Beispiel mit einem primären Amin: 3-Methoxypropylamin

In einem Reaktionsgefäß werden 622 g eines ethoxylierten dreiwertigen Alkohols (Trimethylolpropan) zusammen mit 2 g Schwefelsäure und 529 g Acrylsäure (frisch destilliert) zusammengebracht, danach wird 384 g eines Schleppmittels (Cyclohexan) eingeführt und die entstandene Mischung unter kräftigem Rühren am Rückfluß und Wasserabscheider erhitzt. Nach einer Zeit von 6 - 8 Stunden ist die theoretisch abscheidbare Wassermenge ausgeschieden worden und der Reaktionsansatz wird vom enthaltenen azeotropen Schleppmittel durch Destillation unter vermindertem Druck befreit. Im Reaktionsgefäß befindet sich danach 1020,5 g eines freie Acrylsäure enthaltenden oligomeren Triacrylats. Nach Bestimmung des Gehaltes an freier Acrylsäure durch Titration der Säurezahl (Säurezahl SZ = 15,7) werden 51,3 g (2fache stöchiometrische Menge) von 3-Methoxy-Propylamin unter Zutropfen hinzugefügt. Nach dem Abkühlen der Reaktionsmischung wird das Acrylat abgefüllt und direkt zur Strahlungshärtung von Überzugsmassen auf Holz und Papier verwendet.
Kennwerte: Viskosität 285 mPas, Jodfarbzahl: 1-2, geruchsfrei, lagerstabil bei 60°C über 3 Monate.

### 8. Beispiel mit sekundärem Amin: N-Ethyl-N-isopropanolamin

In einem Reaktionsgefäß werden 622 g eines ethoxylierten dreiwertigen Alkohols (Trimethylolpropan) zusammen mit 2 g Schwefelsäure und 529 g Acrylsäure (frisch destilliert) zusammengebracht, danach wird 384 g Cyclohexan eingeführt und die entstandene Mischung unter kräftigem Rühren am Rückfluß und Wasserabscheider erhitzt. Nach einer Zeit von 6 - 8 Stunden ist die theoretisch abscheidbare Wassermenge ausgeschieden worden und der Reaktionsansatz wird vom enthaltenen azeotropen Schleppmittel durch Destillation unter vermindertem Druck befreit. Im Reaktionsgefäß befindet sich danach 1020,5 g eines freie Acrylsäure enthaltenden oligomeren Triacrylats. Nach Bestimmung des Gehaltes an freier Acrylsäure durch Titration der Säurezahl (SZ = 15,7) werden 46,3 g (2fache stöchiometrische Menge) von N-Ethyl-N-isopropanolamin unter Zutropfen hinzugefügt. Nach dem Abkühlen der Reaktionsmischung wird das Acrylat abgefüllt und direkt zur Strahlungshärtung von Überzugsmassen auf Holz und Papier verwendet.
Kennwerte: Viskosität 130 mPas, Jodfarbzahl: 1 - 2, geruchsfrei, lagerstabil bei 60°C über 3 Monate.

### 9. Beispiel mit einem tertiären Amin: Dimethylisopropylamin

In einem Reaktionsgefäß werden 622 g eines ethoxylierten dreiwertigen Alkohols (Trimethylolpropan) zusammen mit 2 g Schwefelsäure und 529 g Acrylsäure (frisch destilliert) zusammengebracht, danach wird 384 g Cyclohexan eingeführt und die entstandene Mischung unter kräftigem Rühren am Rückfluß und Wasserabscheider erhitzt. Nach einer Zeit von 6 - 8 Stunden ist die theoretisch abscheidbare Wassermenge ausgeschieden worden und der Reaktionsansatz wird vom enthaltenen azeotropen Schleppmittel durch Destillation unter vermindertem Druck befreit. Im Reaktionsgefäß befindet sich danach 1020,5 g eines freie Acrylsäure enthaltenden oligomeren Triacrylats. Nach Bestimmung des Gehaltes an freier Acrylsäure durch Titration der Säurezahl (SZ = 15,7) werden 31,7 g (1,2fache stöchiometrische Menge) von Dimethylisopropylamin unter Zutropfen hinzugefügt. Nach dem Abkühlen der Reaktionsmischung wird das Acrylat abgefüllt und direkt zur Strahlungshärtung von Überzugsmassen auf Holz und Papier verwendet.
Kennwerte: Viskosität 285 mPas, Jodfarbzahl: 1 - 2, geruchsfrei, lagerstabil bei 60°C über 3 Monate.

### Anwendungstechnische Prüfungen

Den erhaltenen (Meth)acrylsäureestern der Beispiele 1 bis 3 wurden jeweils 3 Gew.-% Irgacure^{®} als Photoinitiator zugesetzt. Die (Meth)acrylsäureester wurden mit einem Kastenrakel aufgezogen (Schichtdicke 50 µm) und mit 2 UV-Lampen zu je 80 Watt bestrahlt.

### Reaktivität

Bei der Bestrahlung liegen die Proben auf einem Transportband, welches unter den UV-Lampen durchgeführt wird. Ein Maß für die Reaktivität ist die Geschwindigkeit des Transportbandes, bei der die Proben zu kratzfesten Beschichtungen härten (in m/min.).

### Pendeldämpfung und Erichsentiefung

Bestimmt wurde die Pendeldämpfung nach DIN 53157 (angegeben in Sekunden), welche ein Maß für die Härte einer Beschichtung ist, und die Erichsentiefung nach DIN ISO 1520 (angegeben in Millimetern), welche ein Maß für die Flexibilität, Elastizität einer Beschichtung ist.

### Chemikalienbeständigkeit

Die Chemikalienbeständigkeit wurde bestimmt gemäß DIN 68861 und ist angegeben in Noten von 0 bis 5, wobei niedrige Werte einer guten Chemikalienbeständigkeit entsprechen.

**Tabelle:**

| Ergebnisse der Prüfungen | | | | |
|---|---|---|---|---|
| Beispiel | Pendeldämpfung | Erichsentiefung | Chemikalienbeständigkeit | Reaktivität |
| 1 | 78 | 3,85 | 0,85 | 25 |
| 2 | 106 | 2,9 | 0,85 | 15 |
| 3 | 81 | 3,85 | 0,85 | 15 |

## Patentansprüche

1. Verfahren zur Herstellung von (Meth)acrylsäureestern durch Veresterung von (Meth)acrylsäure mit ein- oder mehrwertigen Alkoholen, dadurch gekennzeichnet, daß keine Umsetzung der restlichen (Meth)acrylsäure mit Epoxiden erfolgt, sondern daß dem Reaktionsgemisch, welches noch (Meth)acrylsäure entsprechend einer Säurezahl von mindestens 5 mg KOH pro 1 g Reaktionsgemisch enthält, mindestens eine Aminoverbindung mit mindestens einer primären, sekundären oder tertiären Aminogruppe, welche mit (Meth)acrylsäure ein Salz bildet, zugesetzt wird.

2. Verfahren gemäß Anspruch 1, dadurch gekennzeichnet, daß die Anzahl der primären, sekundären und tertiären Aminogruppen der Aminoverbindungen mindestens äquimolar zur (Meth)acrylsäure ist.

3. Verfahren gemäß Anspruch 1 oder 2, dadurch gekennzeichnet, daß es sich bei der Aminoverbindung um
C₂ - C₁₀ Alkylamine, welche durch eine C₁ - C₁₀ Alkoxygruppe am Alkylrest substituiert sind, oder
um N-Ethyl-isopropanolamin,
N-Ethyl-cyclohexylamin,
N,N-Dimethyl-isopropanolamin
N-Hydroxyethyl-morpholin,
N-Hydroxyethyl, N-butyl-butylamin,
N-Methyl, -N-isopropanol-isopropanolamin,
N,N-Diethyl-4-amino-pentylamin,
N,N-Diethyl.3-amino-propylamin
handelt.

4. (Meth)acrylsäureester, erhältlich nach einem Verfahren gemäß einem der Ansprüche 1 bis 3.

5. Strahlungshärtbare Massen, enthaltend (Meth)acrylsäureester gemäß Anspruch 4.

6. Verwendung von strahlungshärtbaren Massen gemäß Anspruch 5 zur Herstellung von Beschichtungen.

## Claims

1. A process for preparing (meth)acrylic esters by esterifying (meth)acrylic acid with monohydric or polyhydric alcohols, which comprises not reacting the remaining (meth)acrylic acid with epoxides but instead adding to the reaction mixture still comprising (meth)acrylic acid corresponding to an acid number of at least 5 mg of KOH per 1 g of reaction mixture at least one amino compound having at least one primary, secondary or tertiary amino group which forms a salt with (meth)acrylic acid.

2. A process as claimed in claim 1, wherein the number of the primary, secondary and tertiary amino groups of the amino compounds is at least equimolar to the (meth)acrylic acid.

3. A process as claimed in claim 1 or 2, wherein the amino compound comprises
C₂ - C₁₀ -alkylamines substituted by a C₁ - C₁₀-alkoxy group on the alkyl radical, or
N-ethylisopropanolamine,
N-ethylcyclohexylamine,
N,N-dimethylisopropanolamine,
N-hydroxyethylmorpholine,
N-hydroxyethyl-, N-butyl-butylamine,
N-methyl-N-isopropanol-isopropanolamine,
N,N-diethyl-4-aminopentylamine,
N,N-diethyl-3-aminopropylamine.

4. (Meth)acrylic esters obtainable by a process as claimed in any of claims 1 to 3.

5. Radiation-curable compositions comprising (meth)acrylic esters as claimed in claim 4.

6. The use of radiation-curable compositions as claimed in claim 5 for preparing coatings.

## Revendications

1. Procédé de préparation d'esters d'acide (méth)acrylique par estérification d'acide (méth)acrylique avec des alcools mono- ou polyvalents, caractérisé en ce qu'aucune réaction de l'acide (méth)acrylique résiduel avec des époxydes n'a lieu, mais que l'on ajoute au mélange réactionnel, qui contient encore de l'acide (méth)acrylique correspondant à un indice d'acide d'au moins 5 mg de KOH pour 1 g de mélange réactionnel, au moins un composé amino comportant au moins un groupement amino primaire, secondaire ou tertiaire, qui forme un sel avec l'acide (méth)acrylique.

2. Procédé selon la revendication 1, caractérisé en ce que le nombre des groupements amino primaires, secondaires et tertiaires du composé amino est au moins équimolaire par rapport à l'acide (méth)acrylique.

3. Procédé selon la revendication 1 ou 2, caractérisé en ce que le composé amino est
des alkylamines en C₂-C₁₀, qui sont substituées par un groupement alcoxy en C₁-C₁₀ sur le groupe alkyle, ou
la **N**-éthyl-isopropanolamine,
la **N**-éthyl-cyclohexylamine,
la **N**,**N**-diméthyl-isopropanolamine,
la **N**-hydroxyéthyl-morpholine,
la **N**-hydroxyéthyl-**N**-butyl-butylamine,
la **N**-méthyl-**N**-isopropanol-isopropanolamine,
la **N**,**N**-diéthyl-4-amino-pentylamine,
la **N**,**N**-diéthyl-3-amino-propylamine.

4. Ester d'acide (méth)acrylique obtenu par un procédé selon l'une quelconque des revendications 1 à 3.

5. Masses durcissables par rayonnement contenant un ester (méth)acrylique selon la revendication 4.

6. Utilisation de masses durcissables par rayonnement selon la revendication 5 pour la fabrication de revêtements.
